# EUROPEAN PATENT APPLICATION

(11) **EP 2 055 789 A1**
(43) Date of publication of application: **06.05.2009**
(21) Application number: 07832870.5
(22) Date of filing: 30.11.2007
(51) Int. Cl.: C12Q 1/68, C12N 15/09, C12P 19/34

(54) **PRIMER SET FOR AMPLIFICATION OF CYP2C19 GENE, REAGENT FOR AMPLIFICATION OF CYP2C19 GENE COMPRISING THE SAME, AND USE OF THE SAME**

(30) Priority: 30.11.2006 JP 2006322957; 07.09.2007 JP 2007232613
(71) Applicant: Arkray, Inc., Kyoto-shi, Kyoto 601-8045 (JP)
(72) Inventor: MAJIMA, Satoshi c/o Arkray, Inc.,, Kyoto-shi, Kyoto 601-8045 (JP); YOSHINAGA, Yuki c/o Arkray, Inc.,, Kyoto-shi, Kyoto 601-8045 (JP)
(74) Representative: Behnisch, Werner
(86) International application number: PCT/JP2007/073205
(87) International publication number: WO 2008/066162

(57) **Abstract**

Primer sets for amplifying target regions containing sites to be detected in the CYP2C19 gene by a gene amplification method are provided, wherein the primer sets can amplify the regions specifically. Two pairs of primer sets are used including forward primers consisting of the base sequences of SEQ ID NOs: 12 and 32 as well as reverse primers consisting of the base sequences of SEQ ID NOs: 22 and 48, respectively. The use of these primer sets makes it possible to amplify two target regions including parts where two types of polymorphisms (CYP2C19*2 and CYP2C19*3) of the CYP2C19 gene are generated, respectively, in the same reaction solution at the same time.

[Sequence Table] TF07039-01.ST25.txt

SEQUENCE LISTING

<110> ARKRAY, Inc.

<120> Primer set and agent for amplification of CYP2C19 gene and use thereof

<130> P24714

<150> JP2006-322957
<151> 2006-11-30

<150> JP2007-232613
<151> 2007-09-07

<160> 79

<170> PatentIn version 3.1

<210> 1
<211> 1474
<212> DNA
<213> Homo sapiens

<400> 1
   cttcaccctg tgatcccact ttcatcctgg gctgtgctcc ctgcaatgtg atctgctcca 60
   ttattttcca gaaacgtttc gattataaag atcagcaatt tcttaacttg atggaaaaat 120
   tgaatgaaaa catcaggatt gtaagcaccc cctggatcca ggtaaggcca agttttttgc 180
   ttcctgagaa accacttaca gtcttttttt ctgggaaatc caaaattcta tattgaccaa 240
   gccctgaagt acatttttga atactacagt cttgcctaga cagccatggg gtgaatatct 300
   ggaaaagatg gcaaagttct ttattttatg cacaggaaat gaatatccca atatagatca 360
   ggcttctaag cccattagct ccctgatcag tgttttttcc actaaactcc aaagccctgt 420
   ttctataaag tactttggtg acagccccaa agcgtgctta tatcactcca tggacatcca 480
   ggcactttgg agtcttccat tactcacaag gcttgtcctt caattcacac tttgtcatat 540
   tgtgtgacag aaatatccta atctaaaaga cattatctcc ttcaaggaca gagaatattt 600
   ggaaccacag aagctgccaa gaaacactga atagggcaga ggtgtttgat gtctcagttg 660
   ggattctagc tgatgagaca gctggttagg aatgaaaaaa ttattgtttt tttggtgtat 720
   gaaccataaa cagacatcac acttttaccc tgtgctgaat tggcatgttt tatactctgc 780
   ctaaataata attgtatgat tttacagaag tcatttaact gctctggtgc acagttggaa 840
   tttgaagtat ctttgagccc ctcccacttc taaaatacta attcaatttc agaggctgct 900
   tgatagaaat caatatagca gggactatct ttgtagtatc aatcaggttg tgcaaactct 960
   tttaacctat gctatcatct ccaaaatgtt aatgtagtaa ttcataccat cttatatttc 1020
   aagattgtag agaagaattg ttgtaaaaag taagagaatt aatataaaga tgcttttata 1080
   ctatcaaaag caggtataag tctaggaaat gattatcatc tttgattctc ttgtcagaat 1140
   tttctttctc aaatcttgta taatcagaga attactacac atgtacaata aaaatttccc 1200
   catcaagata tacaatatat tttatttata tttatagttt taaattacaa ccagagcttg 1260
   gcatattgta tctatacctt tattaaatgc ttttaattta ataaattatt gttttctctt 1320
   agatatgcaa taattttccc actatcattg attatttccc gggaacccat aacaaattac 1380
   ttaaaaacct tgcttttatg gaaagtgata ttttggagaa agtaaaagaa caccaagaat 1440
   cgatggacat caacaaccct cgggacttta ttga 1474

<210> 2
<211> 41
<212> DNA
<213> Artificial

<220>
<223> forward primer

<400> 2
ataaattatt gttttctctt agatatgcaa taattttccc a 41

<210> 3
<211> 40
<212> DNA
<213> Artificial

<220>
<223> forward primer

<400> 3
taaattattg ttttctctta gatatgcaat aattttccca 40

<210> 4
<211> 39
<212> DNA
<213> Artificial

<220>
<223> forward primer

<400> 4
aaattattgt tttctcttag atatgcaata attttccca 39

<210> 5
<211> 38
<212> DNA
<213> Artificial

<220>
<223> forward primer

<400> 5
aattattgtt ttctcttaga tatgcaataa ttttccca 38

<210> 6
<211> 37
<212> DNA
<213> Artificial

<220>
<223> forward primer

<400> 6
attattgttt tctcttagat atgcaataat tttccca 37

<210> 7

<211>  36
<212>  DNA
<213>  Artificial

<220>
<223>  forward primer

<400>  7
ttattgtttt ctcttagata tgcaataatt ttccca                                36


<210>  8
<211>  35
<212>  DNA
<213>  Artificial

<220>
<223>  forward primer

<400>  8
tattgttttc tcttagatat gcaataattt tccca                                 35


<210>  9
<211>  34
<212>  DNA
<213>  Artificial

<220>
<223>  forward primer

<400>  9
attgttttct cttagatatg caataatttt ccca                                  34


<210>  10
<211>  33
<212>  DNA
<213>  Artificial

<220>
<223>  forward primer

<400>  10
ttgttttctc ttagatatgc aataattttc cca                                   33


<210>  11
<211>  32
<212>  DNA
<213>  Artificial

<220>
<223>  forward primer

<400>  11
tgttttctct tagatatgca ataattttcc ca                                    32


<210>  12
<211>  31
<212>  DNA
<213>  Artificial

<220>
<223>  forward primer

<400>  12

gttttctctt agatatgcaa taattttccc a                                    31

<210>  13
<211>  30
<212>  DNA
<213>  Artificial

<220>
<223>  forward primer

<400>  13
ttttctctta gatatgcaat aattttccca                                     30

<210>  14
<211>  29
<212>  DNA
<213>  Artificial

<220>
<223>  forward primer

<400>  14
tttctcttag atatgcaata attttccca                                      29

<210>  15
<211>  28
<212>  DNA
<213>  Artificial

<220>
<223>  forward primer

<400>  15
ttctcttaga tatgcaataa ttttccca                                       28

<210>  16
<211>  27
<212>  DNA
<213>  Artificial

<220>
<223>  forward primer

<400>  16
tctcttagat atgcaataat tttccca                                        27

<210>  17
<211>  26
<212>  DNA
<213>  Artificial

<220>
<223>  forward primer

<400>  17
ctcttagata tgcaataatt ttccca                                         26

<210>  18
<211>  25
<212>  DNA
<213>  Artificial

<220>
<223>  forward primer

<400>  18
tcttagatat gcaataattt tccca                                          25


<210>  19
<211>  24
<212>  DNA
<213>  Artificial

<220>
<223>  reverse primer

<400>  19
tcccgagggt tgttgatgtc catc                                           24


<210>  20
<211>  23
<212>  DNA
<213>  Artificial

<220>
<223>  reverse primer

<400>  20
cccgagggtt gttgatgtcc atc                                            23


<210>  21
<211>  22
<212>  DNA
<213>  Artificial

<220>
<223>  reverse primer

<400>  21
ccgagggttg ttgatgtcca tc                                             22


<210>  22
<211>  21
<212>  DNA
<213>  Artificial

<220>
<223>  reverse primer

<400>  22
cgagggttgt tgatgtccat c                                              21


<210>  23
<211>  20
<212>  DNA
<213>  Artificial

<220>
<223>  reverse primer

<400>  23
gagggttgtt gatgtccatc                                                20


<210>  24

<211>  19
<212>  DNA
<213>  Artificial

<220>
<223>  reverse primer

<400>  24
agggttgttg atgtccatc                                                    19


<210>  25
<211>  37
<212>  DNA
<213>  Artificial

<220>
<223>  forward primer

<400>  25
cttgatggaa aaattgaatg aaaacatcag gattgta                                37


<210>  26
<211>  36
<212>  DNA
<213>  Artificial

<220>
<223>  forward primer

<400>  26
ttgatggaaa aattgaatga aaacatcagg attgta                                 36


<210>  27
<211>  35
<212>  DNA
<213>  Artificial

<220>
<223>  forward primer

<400>  27
tgatggaaaa attgaatgaa aacatcagga ttgta                                  35


<210>  28
<211>  34
<212>  DNA
<213>  Artificial

<220>
<223>  forward primer

<400>  28
gatggaaaaa ttgaatgaaa acatcaggat tgta                                   34


<210>  29
<211>  33
<212>  DNA
<213>  Artificial

<220>
<223>  forward primer

<400>  29

atggaaaaat tgaatgaaaa catcaggatt gta                                    33

<210>  30
<211>  32
<212>  DNA
<213>  Artificial

<220>
<223>  forward primer

<400>  30
tggaaaaatt gaatgaaaac atcaggattg ta                                     32

<210>  31
<211>  31
<212>  DNA
<213>  Artificial

<220>
<223>  forward primer

<400>  31
ggaaaaattg aatgaaaaca tcaggattgt a                                      31

<210>  32
<211>  30
<212>  DNA
<213>  Artificial

<220>
<223>  forward primer

<400>  32
gaaaaattga atgaaaacat caggattgta                                        30

<210>  33
<211>  29
<212>  DNA
<213>  Artificial

<220>
<223>  forward primer

<400>  33
aaaaattgaa tgaaaacatc aggattgta                                         29

<210>  34
<211>  28
<212>  DNA
<213>  Artificial

<220>
<223>  forward primer

<400>  34
aaaattgaat gaaaacatca ggattgta                                          28

<210>  35
<211>  27
<212>  DNA
<213>  Artificial

<220>
<223>  forward primer

<400>  35
aaattgaatg aaaacatcag gattgta                                          27


<210>  36
<211>  26
<212>  DNA
<213>  Artificial

<220>
<223>  forward primer

<400>  36
aattgaatga aaacatcagg attgta                                           26


<210>  37
<211>  25
<212>  DNA
<213>  Artificial

<220>
<223>  forward primer

<400>  37
attgaatgaa aacatcagga ttgta                                            25


<210>  38
<211>  24
<212>  DNA
<213>  Artificial

<220>
<223>  forward primer

<400>  38
ttgaatgaaa acatcaggat tgta                                             24


<210>  39
<211>  23
<212>  DNA
<213>  Artificial

<220>
<223>  forward primer

<400>  39
tgaatgaaaa catcaggatt gta                                              23


<210>  40
<211>  30
<212>  DNA
<213>  Artificial

<220>
<223>  reverse primer

<400>  40
tcaaaaatgt acttcagggc ttggtcaata                                       30


<210>  41

<211>  29
<212>  DNA
<213>  Artificial

<220>
<223>  reverse primer

<400>  41
caaaaatgta cttcagggct tggtcaata                                         29


<210>  42
<211>  28
<212>  DNA
<213>  Artificial

<220>
<223>  reverse primer

<400>  42
aaaaatgtac ttcagggctt ggtcaata                                          28


<210>  43
<211>  27
<212>  DNA
<213>  Artificial

<220>
<223>  reverse primer

<400>  43
aaaatgtact tcagggcttg gtcaata                                           27


<210>  44
<211>  26
<212>  DNA
<213>  Artificial

<220>
<223>  reverse primer

<400>  44
aaatgtactt cagggcttgg tcaata                                            26


<210>  45
<211>  25
<212>  DNA
<213>  Artificial

<220>
<223>  reverse primer

<400>  45
aatgtacttc agggcttggt caata                                             25


<210>  46
<211>  24
<212>  DNA
<213>  Artificial

<220>
<223>  reverse primer

<400>  46

atgtacttca gggcttggtc aata                                          24

<210>  47
<211>  23
<212>  DNA
<213>  Artificial

<220>
<223>  reverse primer

<400>  47
tgtacttcag ggcttggtca ata                                           23

<210>  48
<211>  22
<212>  DNA
<213>  Artificial

<220>
<223>  reverse primer

<400>  48
gtacttcagg gcttggtcaa ta                                            22

<210>  49
<211>  21
<212>  DNA
<213>  Artificial

<220>
<223>  reverse primer

<400>  49
tacttcaggg cttggtcaat a                                             21

<210>  50
<211>  20
<212>  DNA
<213>  Artificial

<220>
<223>  reverse primer

<400>  50
acttcagggc ttggtcaata                                               20

<210>  51
<211>  19
<212>  DNA
<213>  Artificial

<220>
<223>  reverse primer

<400>  51
cttcagggct tggtcaata                                                19

<210>  52
<211>  18
<212>  DNA
<213>  Artificial

<220>
<223>  reverse primer

<400>  52
ttcagggctt ggtcaata                                                    18


<210>  53
<211>  28
<212>  DNA
<213>  Artificial

<220>
<223>  probe

<400>  53
cattgattat ttcccaggaa cccataac                                         28


<210>  54
<211>  27
<212>  DNA
<213>  Artificial

<220>
<223>  probe

<400>  54
attgattatt tcccaggaac ccataac                                          27


<210>  55
<211>  26
<212>  DNA
<213>  Artificial

<220>
<223>  probe

<400>  55
ttgattattt cccaggaacc cataac                                           26


<210>  56
<211>  25
<212>  DNA
<213>  Artificial

<220>
<223>  probe

<400>  56
tgattatttc ccaggaaccc ataac                                            25


<210>  57
<211>  24
<212>  DNA
<213>  Artificial

<220>
<223>  probe

<400>  57
gattatttcc caggaaccca taac                                             24


<210>  58

<211>  23
<212>  DNA
<213>  Artificial

<220>
<223>  probe

<400>  58
attatttccc aggaacccat aac                                              23


<210>  59
<211>  22
<212>  DNA
<213>  Artificial

<220>
<223>  probe

<400>  59
ttatttccca ggaacccata ac                                               22


<210>  60
<211>  21
<212>  DNA
<213>  Artificial

<220>
<223>  probe

<400>  60
tatttcccag gaacccataa c                                                21


<210>  61
<211>  20
<212>  DNA
<213>  Artificial

<220>
<223>  probe

<400>  61
atttcccagg aacccataac                                                  20


<210>  62
<211>  19
<212>  DNA
<213>  Artificial

<220>
<223>  probe

<400>  62
tttcccagga acccataac                                                   19


<210>  63
<211>  18
<212>  DNA
<213>  Artificial

<220>
<223>  probe

<400>  63

ttcccaggaa cccataac                                                    18


<210>  64
<211>  17
<212>  DNA
<213>  Artificial

<220>
<223>  probe

<400>  64
tcccaggaac ccataac                                                     17


<210>  65
<211>  16
<212>  DNA
<213>  Artificial

<220>
<223>  probe

<400>  65
cccaggaacc cataac                                                      16


<210>  66
<211>  25
<212>  DNA
<213>  Artificial

<220>
<223>  probe

<400>  66
   caccccctga atccaggtaa ggcca                                           25


<210>  67
<211>  24
<212>  DNA
<213>  Artificial

<220>
<223>  probe

<400>  67
   caccccctga atccaggtaa ggcc                                            24


<210>  68
<211>  23
<212>  DNA
<213>  Artificial

<220>
<223>  probe

<400>  68
   caccccctga atccaggtaa ggc                                             23


<210>  69
<211>  22
<212>  DNA
<213>  Artificial

<220>
<223>  probe

<400>  69
   caccccctga atccaggtaa gg                                              22

<210>  70
<211>  21
<212>  DNA
<213>  Artificial

<220>
<223>  probe

<400>  70
   caccccctga atccaggtaa g                                               21

<210>  71
<211>  20
<212>  DNA
<213>  Artificial

<220>
<223>  probe

<400>  71
   caccccctga atccaggtaa                                                 20

<210>  72
<211>  19
<212>  DNA
<213>  Artificial

<220>
<223>  probe

<400>  72
   caccccctga atccaggta                                                  19

<210>  73
<211>  18
<212>  DNA
<213>  Artificial

<220>
<223>  probe

<400>  73
   caccccctga atccaggt                                                   18

<210>  74
<211>  19
<212>  DNA
<213>  Artificial

<220>
<223>  probe

<400>  74
ccctgaatcc aggtaaggc                                                   19

<210>  75

<211>  18
<212>  DNA
<213>  Artificial

<220>
<223>  probe

<400>  75
ccctgaatcc aggtaagg                                                    18


<210>  76
<211>  17
<212>  DNA
<213>  Artificial

<220>
<223>  probe

<400>  76
ccctgaatcc aggtaag                                                     17


<210>  77
<211>  16
<212>  DNA
<213>  Artificial

<220>
<223>  probe

<400>  77
ccctgaatcc aggtaa                                                      16


<210>  78
<211>  15
<212>  DNA
<213>  Artificial

<220>
<223>  probe

<400>  78
ccctgaatcc aggta                                                       15


<210>  79
<211>  14
<212>  DNA
<213>  Artificial

<220>
<223>  probe

<400>  79
ccctgaatcc aggt                                                        14

## Description

### Technical Field

The present invention relates to primer sets for amplifying the CYP2C19 gene, reagents for amplifying the CYP2C19 gene containing the same, and the uses thereof.

### Background Art

Cytochrome P450 is an enzyme group that is classified into a super family and includes many subfamilies (for example, CYP1A, CYP1B, CYP2C, CYP2D, CYP2E, CYP3A, etc.). Among them, CYP2C19, an isozyme of a human CYP2C subfamily, is known as an enzyme that is involved in a drug-metabolizing. Further, a mutation of a gene coding CYP2C19 (CYP2C19 gene) is found in poor metabolizers (PMs) whose metabolism of (S)-mephenytoin (S-Mep), a substrate drug of CYP2C19 (antiepileptic drug), is very low. Known examples of important genetic polymorphisms involved in the PMs include CYP2C19*2 and CYP2C19*3. The former is a mutation in which guanine (position 681) in exon 5 is changed to adenine. This mutation causes the splicing defect and changes the translation start site of the gene information. The latter is a mutation in which guanine (position 636) in exon 4 is changed to adenine and the translation is interrupted at the mutation site because of change to the stop codon. With respect to Asian including Japanese, PMs can be judged with almost 100% accuracy by analyzing these two polymorphisms. Further, since about 85% of Caucasian PMs have these polymorphisms, they are considered as major polymorphisms regardless of race.

Besides the aforementioned PMs, these polymorphisms (CYP2C19 *2 and CYP2C19 *3) of the CYP2C19 gene affect, for example, the metabolism from diazepam, a hypnotic sedative, to N-demethylator (nordiazepam). Further, these polymorphisms affect the metabolism of the antiepileptic drug phenytoin (PHT), the tricyclic antidepressant drug imipramine, the hypnotic sedative hexobarbital, the malaria medicine proguanil, the muscle relaxant agent carisoprodol, and proton pump inhibitors (PPI) such as omeprazole (OPZ), lansoprazole, rabeprazole, etc. Particularly, with respect to OPZ, which is one type of PPI, it is reported that these polymorphisms (CYP2C19 *2 and CYP2C19 *3) of the CYP2C19 gene improve, as a drug response, an eradication rate of Helicobacter pylori bacteria by a combination use of OPZ, clarithromycin, and amoxicillin. Accordingly, examination of two polymorphisms CYP2C19 *2 and CYP2C19 *3 with respect to the CYP2C19 gene is very important to predict side effects from the drug and to determine the dosing condition that shows excellent effect.

On the other hand, detection of a point mutation, a so-called single nucleotide polymorphism (SNP), is employed widely as a method of analyzing, at the gene level, for example, the causes of all types of diseases and the individual differences in disease liability (susceptibility to diseases) and in drug action. Examples of the common methods of detecting a point mutation include: (1) a direct sequencing method in which the region corresponding to a sequence to be detected in a target DNA of a sample is amplified by a polymerase chain reaction (PCR) and all the gene sequences are analyzed, (2) a RFLP analysis in which the region corresponding to a sequence to be detected in a target DNA of a sample is amplified by PCR, the amplification product thus obtained is cut with a restriction enzyme whose cleaving action differs depending on the presence or absence of the target mutation in the sequence to be detected and is then electrophoresed, and thereby typing is performed, and (3) the ASP-PCR method in which PCR is performed using a primer with a target mutation located at the 3'-end region and the mutation is judged depending on the presence or absence of amplification.

However, since these methods require, for example, purification of DNA extracted from a sample, electrophoresis, and a treatment with a restriction enzyme, they take time and cost. Furthermore, after PCR is performed, it is necessary to open the reaction container once. Accordingly, there is a possibility that the amplification product may contaminate the next reaction system and thereby the analysis accuracy may be deteriorated. Moreover, since it is difficult to automate, multiple samples cannot be analyzed. Further, the aforementioned ASP-PCR method (3) is less specific, which also is a problem.

Because of these problems, recently, a method of analyzing the melting temperature (Tm) of double-stranded nucleic acid formed of a probe and target nucleic acid is used as a method of detecting a point mutation. Since such a method is performed through, for example, Tm analysis or analysis of the melting curve of the double strand, it is referred to as melting curve analysis. This method is described below. That is, first, a probe complementary to a sequence to be detected containing a target point mutation is used to form a hybrid (double-stranded DNA) between the aforementioned probe and a target single-stranded DNA contained in a detection sample. Subsequently, this hybridization product is heat-treated, and dissociation (melting) of the hybrid accompanying the temperature rise is detected by a change in a signal such as absorbance. The Tm value is then determined based on the result of the detection and the presence or absence of any point mutation is judged accordingly. The higher the homology of the hybridization product, the higher the Tm value, and the lower the homology, the lower the Tm value. Therefore the Tm value (reference value for assessment) is determined beforehand with respect to the hybridization product between the sequence to be detected containing a point mutation and a probe complementary thereto, and then the Tm value (measured value) of the hybridization product between the target single-stranded DNA contained in the detection sample and the aforementioned probe is measured. When the measured value is comparable to the reference value, it is considered as matching, that is, it can be judged that a point mutation is present in the target DNA. On the other hand, when the measured value is lower than the reference value, it is considered as mismatching, that is, it can be judged that no point mutation is present in the target DNA. Furthermore, according to this method, it also is possible to automate gene analysis.

However, such a detection method using Tm analysis also has a problem in that a region including a site to be detected must be able to be amplified specifically and efficiently in PCR. Particularly, cytochrome P450 is the enzyme group classified into the super family as described above and isozymes with very high homology are present in molecular species that belong to the same subfamily (CYP2C subfamily) therewith and the sequences for coding them also are very similar to one another. Accordingly, there is a possibility that genes coding isozymes other than CYP2C19 also are amplified in PCR. Furthermore, when other isozyme-coding genes also have been amplified as described above, it may cause a decrease in the reliability of the analysis result in the analysis of a particular polymorphism (CYP2C19*2 or CYP2C19*3) of the CYP2C19 gene (Nonpatent Documents 1 and 2). Moreover, as described above, since analysis of one sample is accompanied by a considerable amount of time and energy, it is not practical to analyze multiple samples, which also is a problem.
[Nonpatent Document 1] PMID: 8195181 J Biol Chem. 1994 Jun 3; 269(22): 15419-22.
[Nonpatent Document 2] PMID: 7969038 Mol Pharmacol. 1994 Oct; 46(4): 594-8.

### Disclosure of Invention

Hence, the present invention is intended to provide primer sets for specifically amplifying a target region in the CYP2C19 gene by a gene amplification method.

In order to achieve the aforementioned object, a primer set of the present invention is a primer set for amplifying the CYP2C19 gene by a gene amplification method, wherein the primer set includes at least one of the following primer sets (1) and (2):
Primer set (1):
   a primer set of a pair of primers including a forward primer composed of the following oligonucleotide (F1) and a reverse primer composed of the following oligonucleotide (R1):
      (F1): at least one oligonucleotide having a sequence identical to that of a region extending from adenine (A) at base 1341 to be considered as the first base to any one of the 25^{th} to 41^{st} bases in the direction toward the 5' end in the base sequence of SEQ ID NO: 1, with the adenine (A) being the 3' end, (R1): at least one oligonucleotide complementary to a region extending from guanine (G) at base 1442 to be considered as the first base to any one of the 19^{th} to 24^{th} bases in the direction toward the 3' end in the base sequence of SEQ ID NO: 1, with cytosine (C) complementary to the guanine (G) at base 1442 being the 3' end, and
Primer set (2):
   a primer set of a pair of primers including a forward primer composed of the following oligonucleotide (F2) and a reverse primer composed of the following oligonucleotide (R2):
      (F2): at least one oligonucleotide having a sequence identical to that of a region extending from adenine (A) at base 143 to be considered as the first base to any one of the 23^{rd} to 37^{th} bases in the direction toward the 5' end in the base sequence of SEQ ID NO: 1, with the adenine (A) being the 3' end, and
      (R2): at least one oligonucleotide complementary to a region extending from thymine (T) at base 231 to be considered as the first base to any one of the 18^{th} to 30^{th} bases in the direction toward the 3' end in the base sequence of SEQ ID NO: 1, with adenine (A) complementary to the thymine (T) at base 231 being the 3' end.

A reagent for amplifying a gene of the present invention is a reagent for amplifying the CYP2C19 gene by a gene amplification method, wherein the reagent includes the primer set for amplifying the CYP2C19 gene of the present invention.

A method of manufacturing an amplification product of the present invention is a method of manufacturing an amplification product of the CYP2C19 gene by a gene amplification method, wherein the method includes the following step (I):
(I) amplifying the CYP2C19 gene in a reaction solution using a primer set for amplifying the CYP2C19 gene according to the present invention, with nucleic acid contained in a sample being used as a template.

A polymorphism analysis method of the present invention is a method of analyzing a polymorphism of a site to be detected in the CYP2C19 gene, wherein the method includes the following steps (i) to (iv):
(i) amplifying a region including a site to be detected in the CYP2C19 gene in a reaction solution by a method of manufacturing an amplification product of the present invention,
(ii) preparing a reaction solution that contains the amplification product obtained in step (i) and a probe capable of hybridizing to the site to be detected,
(iii) measuring signal values that indicate melting states of a hybridization product between the amplification product and the probe while changing the temperature of the reaction solution, and
(iv) determining a polymorphism of the site to be detected from a change in the signal values accompanying a change in the temperature.

### Effect of Invention

The primer set of the present invention makes it possible to amplify a target region in a reaction solution specifically and efficiently, with the target region including the site where a polymorphism to be detected (CYP2C19*2 or CYP2C19*3) is generated in the CYP2C19 gene. Accordingly, the time and cost can be reduced, which is different from the conventional methods described above. Furthermore, as described above, since a region including a site to be detected where a specific polymorphism of the CYP2C19 gene is generated can be amplified specifically, for example, further the use of a probe complementary to a sequence to be detected including the site to be detected makes it possible to perform Tm analysis by directly using the aforementioned reaction solution to type the polymorphism. Moreover, since amplification of the target region and typing of the polymorphism can be performed with one reaction solution, it is also possible to automate the operation. Since the use of the primer set of the present invention allows a pretreatment to be omitted even in the case of, for example, a contaminated sample (for instance, whole blood or oral mucosa etc.), the amplification reaction can be carried out quicker and more simply. Furthermore, since the use of the primer set of the present invention allows the amplification reaction to be carried out with higher amplification efficiency as compared to the conventional case, the amplification reaction time also can be shortened. Thus, according to the primer set of the present invention and a reagent including the same as well as the method of manufacturing an amplification product, in which the primer set and the reagent are used, polymorphisms in the CYP2C19 gene can be analyzed quickly and simply, and it therefore can be said that they are very effective in the field of medicine.

### Brief Description of Drawings

FIG. 1 shows graphs indicating the results of Tm analysis in Example 1 of the present invention.
FIG. 2 shows graphs indicating the results of Tm analysis in Example 2 of the present invention.

### Best Mode for Carrying Out the Invention

### < Primer set for amplifying CYP2C19 gene>

As described above, the primer set for amplifying the CYP2C19 gene of the present invention is characterized by including at least one of the aforementioned primer sets (1) and (2). The inclusion of at least one of the primer sets makes it possible, for example, to amplify specifically a specific target region in the CYP2C19 gene.

The primer set for amplifying the CYP2C19 gene of the present invention may include, for example, one of the aforementioned primer sets (1) and (2) or may include both of the primer sets (1) and (2). As described later, the target region that can be amplified specifically with the primer set (1) is a region including a site where the polymorphism CYP2C19*2 is generated in the CYP2C19 gene; and the target region that can be amplified specifically with the primer set (2) is a region including a site where the polymorphism CYP2C19*3 is generated in the CYP2C19 gene.

As described above, since these two types of polymorphisms in the CYP2C19 gene are known as polymorphisms that affect drug metabolism, it is considered to be important to examine not only one of them but all of the two types of polymorphisms. However, the conventional methods have a problem in that a plurality of sequences cannot be analyzed in one reaction system. Conceivably, as described above, this is because the many isozymes exist in a CYP2C and thereby genes coding isozymes other than CYP2C19 also are amplified in PCR. Accordingly, in order to examine both of the two types of polymorphisms (CYP2C19*2 and CYP2C19*3) in the CYP2C19 gene, it is necessary that the regions including the sites where the respective polymorphisms are generated are amplified in separate reaction systems, respectively, and the resultant amplification products are analyzed separately. Thus, with the conventional methods, it is very difficult to use only the CYP2C19 gene selected from the CYP2C genes as a template and to amplify specifically only two types of target regions including the sites where polymorphisms are generated, respectively, in the CYP2C19 gene. Furthermore, since such analysis of even one sample is accompanied by a considerable amount of work, there is a problem in that the analysis of multiple samples is not practical. On the contrary, according to the primer set for amplifying the CYP2C19 gene of the present invention, even when both of the two types of the primer sets (1) and (2) are included, the respective target regions can be amplified in the same reaction solution simultaneously and specifically. Accordingly, the time and cost can be reduced, which is different from the aforementioned conventional methods. Furthermore, since two target regions are amplified specifically in the same reaction solution as described above, for example, the use of a probe complementary to a sequence to be detected in each target region makes it possible to perform Tm analysis directly using the aforementioned reaction solution to type each of the two types of polymorphisms. As described above, since two types of polymorphisms in the CYP2C19 gene can be analyzed in the same reaction solution, it is suitable for the primer set for amplifying the CYP2C19 gene of the present invention not only to include one of the primer sets (1) and (2) but also to include both of them. When not only one target region but also two target regions are amplified simultaneously using such a CYP2C19 gene primer set, polymorphisms in the CYP2C19 gene can be analyzed more efficiently as compared to the conventional cases.

Hereinafter, a forward primer also may be referred to as "F primer" and a reverse primer as "R primer".

As described above, the primer set (1) is a primer set of a pair of primers including a forward primer composed of the following oligonucleotide (F1) and a reverse primer composed of the following oligonucleotide (R1):
(F1): at least one oligonucleotide having a sequence identical to that of a region extending from adenine (A) at base 1341 to be considered as the first base to any one of the 25^{th} to 41^{st} bases in the direction toward the 5' end in the base sequence of SEQ ID NO: 1, with the adenine (A) being the 3' end, and
(R1): at least one oligonucleotide complementary to a region extending from guanine (G) at base 1442 to be considered as the first base to any one of the 19^{th} to 24^{th} bases in the direction toward the 3' end in the base sequence of SEQ ID NO: 1, with cytosine (C) complementary to the guanine (G) at base 1442 being the 3' end.

The base sequence indicated in SEQ ID NO: 1 is a genome DNA sequence including exon 4 and exon 5 of the CYP2C19 gene in human chromosome 10, and a region from base 1 to 161 in SEQ ID NO: 1 indicates exon 4, a region from base 1323 to 1449 in SEQ ID NO: 1 indicates exon 5, and a region therebetween indicates intron.

The primer set (1) is a primer set for amplifying a DNA strand including a region from base 1342 to base 1441 in SEQ ID NO: 1, as well as a strand complementary thereto. Base 1361 in this region (i.e. base 1361 in SEQ ID NO: 1) is known for the presence of a point mutation (1361G, 1361A) that affects the function of CYP2C19, and the polymorphism thereof is CYP2C19*2 described above. In the present invention, the polymorphism of this site can be indicated as 1361G/G or 1361A/A in the case of homozygote and as 1361G/A in the case of heterozygote. Further, since base 1361 in SEQ ID NO: 1 corresponds to base 681 of exon 5 in mRNA of the CYP2C19 gene, as the polymorphism CYP2C19*2, it can be indicated, for example, as 681G/G, 681A/A, or 681G/A. Hereinafter, this primer set (1) also may be referred to as a "primer set for CYP2C19*2". When only the polymorphism CYP2C19*2 is to be analyzed, it is sufficient to use only the primer set for CYP2C19*2.

In the present invention, the F1 primer and R1 primer of the primer set (1) can be any primers, as long as the base located at the 3' end that serves to determine the site from which DNA polymerase starts amplification satisfies the aforementioned condition. Fixation of the base located at the 3' end of each primer in this manner makes it possible to prevent sufficiently the primer set (1) from being bound to, for example, another similar isozyme gene (for example, CYP2C8 gene, CYP2C9 gene , CYP2C17 gene, or CYP2C18 gene).

As described above, since the F1 primer and R1 primer each can be any primer as long as the base located at the 3' end is fixed, the length itself of each primer is not particularly limited and can be adjusted suitably to be common length. The length of the primers is, for example, in the range of 13- to 50-mers, preferably 14- to 45-mers, and more preferably 15- to 40-mers. Specifically, it is preferable that the F1 primer be at least one oligonucleotide having a sequence identical to that of a region extending from adenine (A) at base 1341 to be considered as the first base to any one of the 25^{th} to 41^{st} bases (preferably the 27^{th} to 38^{th} bases and more preferably the 29^{th} to 34^{th} bases) in the direction toward the 5' end in the base sequence of SEQ ID NO: 1. Furthermore, it is preferable that the R1 primer be at least one oligonucleotide complementary to a region extending from guanine (G) at base 1442 to be considered as the first base to any one of the 19^{th} to 24^{th} bases (preferably the 20^{th} to 23^{rd} bases and more preferably the 20^{th} to 22^{nd} bases) in the direction toward the 3' end in the base sequence of SEQ ID NO: 1. Since each 3' end of the F1 primer and the R1 primer is fixed, the region to be elongated from the primer is, for example, a region from base 1342 to base 1441 in SEQ ID NO: 1 as described above. However, the length of the whole amplification product obtained varies according to the length of the primer to be used.

Furthermore, it is not necessary for the R1 primer and the F1 primer to be oligonucleotides perfectly complementary to the base sequence indicated in SEQ ID NO: 1 and to the strand complementary to the base sequence, respectively. In other words, the part excluding the base located at the 3' end in each primer may be different in one to five bases from that of a perfectly complementary oligonucleotide.

Specific examples of the F1 primer and the R1 primer are indicated below but the present invention is not limited thereto. The combination of these F1 primer and R1 primer is not limited by any means. Specifically, however, a primer set (1') is particularly preferable, which includes a F1' primer composed of oligonucleotide of SEQ ID NO: 3 or SEQ ID NO: 12, and a R1' primer composed of oligonucleotide of SEQ ID NO: 20 or SEQ ID NO: 22. "Tm (°C)" indicated below in the table is Tm (°C) obtained when each sequence indicated below in the table was hybridized with the sequence perfectly complementary thereto. The "Tm (°C)" is a value calculated by using MELTCALC software (http://www.meltcalc.com/), with parameters including an oligonucleotide concentration of 0.2 µM and a sodium equivalent (Na eq.) of 50 mM (the same applies below). The Tm value can be calculated by using, for example, conventionally known MELTCALC software (http://www.meltcalc.com/) or also can be determined by the nearest neighbor method (the same applies below).

**[Table 1]**

| Primer | Sequence | Tm(°C) | SEQ ID NO. |
|---|---|---|---|
| F1 Primer for CYP2C19*2 | 5'-ataaattattgttttctcttagatatgcaataattttccca-3' | 56.7 | 2 |
| | 5"taaattattgttttctcttagatatgcaataattttccca-3' | 56.6 | 3 |
| | 5'-aaattattgttttctcttagatatgcaataattttccca-3' | 56.8 | 4 |
| | 5'-aattattgttttctcttagatatgcaataattttccca-3' | 56.6 | 5 |
| | 5'-attattgttttctcttagatatgcaataattttccca-3' | 56.3 | 6 |
| | 5'-ttattgttttctcttagatatgcaataattttccca-3' | 56.2 | 7 |
| | 5'-tattgttttctcttagatatgcaataattttccca-3' | 55.9 | 8 |
| | 5'-attgttttctcttagatatgcaataattttccca-3' | 56.1 | 9 |
| | 5'-ttgttttctcttagatatgcaataattttccca-3' | 56 | 10 |
| | 5'-tgttttctcttagatatgcaataattttccca-3' | 55.7 | 11 |
| | 5'-gttttctcttagatatgcaataattttccca-3' | 54.6 | 12 |
| | 5'-ttttctcttagatatgcaataattttccca-3' | 53.7 | 13 |
| | 5'-tttctcttagatatgcaataattttccca-3' | 53.3 | 14 |
| | 5'-ttctcttagatatgcaataattttccca-3' | 52.8 | 15 |
| | 5'-tetettagatatgcaataattttccca-3' | 52.3 | 16 |
| | 5'-ctcttagatatgcaataattttccca-3' | 51.2 | 17 |
| | 5'-tcttagatatgcaataattttccca-3' | 50.2 | 18 |
| R1 Primer for CYP2C19*2 | 5'-tcccgagggttgttgatgtccatc-3' | 60.1 | 19 |
| | 5'-cccgagggttgttgatgtccatc-3' | 59.1 | 20 |
| | 5'-ccgagggttgttgatgtccatc-3' | 57.1 | 21 |
| | 5'-cgagggttgttgatgtccatc-3' | 55 | 22 |
| | 5'-gagggttgttgatgtccatc-3' | 52.2 | 23 |
| | 5'-agggttgttgatctccatc-3' | 51 | 24 |

Next, as described above, the primer set (2) is a primer set of a pair of primers including a forward primer composed of the following oligonucleotide (F2) and a reverse primer composed of the following oligonucleotide (R2):
(F2): at least one oligonucleotide having a sequence identical to that of a region extending from adenine (A) at base 143 to be considered as the first base to any one of the 23^{rd} to 37^{th} bases in the direction toward the 5' end in the base sequence of SEQ ID NO: 1, with the adenine (A) being the 3' end, and
(R2): at least one oligonucleotide complementary to a region extending from thymine (T) at base 231 to be considered as the first base to any one of the 18^{th} to 30^{th} bases in the direction toward the 3' end in the base sequence of SEQ ID NO: 1, with adenine (A) complementary to the thymine (T) at base 231 being the 3' end.

The primer set (2) is a primer set for amplifying a DNA strand including a region from base 144 to base 230 in SEQ ID NO: 1 as well as a strand complementary thereto. Base 155 in this region (i.e. base 155 in SEQ ID NO: 1) is known for the presence of a point mutation (155G, 155A) that affects the function of CYP2C19, and the polymorphism thereof is CYP2C19*3 described above. In the present invention, the polymorphism of this site can be indicated as 155G/G or 155A/A in the case of homozygote and as 155G/A in the case of heterozygote. Since base 155 in SEQ ID NO: 1 corresponds to base 636 of exon 4 in mRNA of the CYP2C19 gene, as the polymorphism CYP2C19*3, it can be indicated, for example, as 636G/G, 636A/A, or 636G/A. Hereinafter, this primer set (2) also may be referred to as a "primer set for CYP2C19*3". When only the polymorphism CYP2C19*3 is to be analyzed, it is sufficient to use only the primer set for CYP2C19*3.

From the same reason as that described with respect to the primer set (1), in the present invention, the F2 primer and the R2 primer of the primer set (2) can be any primers, as long as the base located at the 3' end that serves to determine the site from which DNA polymerase starts amplification satisfies the aforementioned condition. Accordingly, the length itself of the F2 primer and the R2 primer is not particularly limited and can be, for example, as described above. Specifically, it is preferable that the F2 primer be at least one oligonucleotide having a sequence identical to that of a region extending from adenine (A) at base 143 to be considered as the first base to any one of the 23^{rd} to 37^{th} bases (preferably the 25^{th} to 35^{th} bases and more preferably the 27^{th} to 33^{rd} bases) in the direction toward the 5' end in the base sequence of SEQ ID NO: 1. Furthermore, it is preferable that the R2 primer be at least one oligonucleotide complementary to a region extending from thymine (T) at base 231 to be considered as the first base to any one of the 18^{th} to 30^{th} bases (preferably the 19^{th} to 27^{th} bases and more preferably the 20^{th} to 25^{th} bases) in the direction toward the 3' end in the base sequence of SEQ ID NO: 1.

Furthermore, it is not necessary for the R2 primer and the F2 primer to be oligonucleotides perfectly complementary to the base sequence indicated in SEQ ID NO: 1 and to the strand complementary to the base sequence, respectively. In other words, the part excluding the base located at the 3' end in each primer may be different in one to five bases from that of a perfectly complementary oligonucleotide.

Specific examples of the F2 primer and the R2 primer are indicated below but the present invention is not limited thereto. The combination of these F2 primer and R2 primer is not limited by any means. Specifically, however, a primer set (2'), which includes a F2' primer composed of oligonucleotide of SEQ ID NO: 27 or SEQ ID NO: 32, and a R2' primer composed of oligonucleotide of SEQ ID NO: 40 or SEQ ID NO: 48, is particularly preferable.

**[Table 2]**

| Primer | Sequence | Tm(°C) | SEQ (°C) NO. |
|---|---|---|---|
| F2 Primer for CYP2C19*3 | 5'-cttgatggaaaaattgaatgaaaacatcaggattgta-3' | 58.8 | 25 |
| | 5'-ttgatggaaaaattgaatgaaaacatcaggattgta-3' | 58.3 | 26 |
| | 5'-tgatggaaaaattgaatgaaaacatcaggattgta-3' | 58.1 | 27 |
| | 5'-gatggaaaaattgaatgaaaacatcaggattgta-3' | 57.2 | 28 |
| | 5'-atggaaaaattgaatgaaaacatcaggattgta-3' | 56.7 | 29 |
| | 5'-tggaaaaattgaatgaaaacatcaggattgta-3' | 56.5 | 30 |
| | 5'-ggaaaaattgaatgaaaacatcaggattgta-3' | 55.5 | 31 |
| | 5'-gaaaaattgaatgaaaacatcaggattgta-3' | 54 | 32 |
| | 5'-aaaaattgaatgaaaacateaggattgta-3' | 53.2 | 33 |
| | 5'-aaaattgaatgaaaacatcaggattgta-3' | 52.7 | 34 |
| | 5'-aaattgaatgaaaacatcaggattgta-3' | 52.2 | 35 |
| | 5'-aattgaatgaaaacatcaggattgta-3' | 51.7 | 36 |
| | 5'-attgaatgaaaacatcaggattgta-3' | 51.1 | 37 |
| | 5'-ttgaatgaaaacatcaggattgta-3' | 50.7 | 38 |
| | 5'-tgaatgaaaacatcaggattgta-3' | 50 | 39 |
| R2 Primer for CYP2C19*3 | 5'-tcaaaaatgtacttcagggcttggtcaata-3' | 58.6 | 40 |
| | 5'-caaaaatgtacttcagggcttggtcaata-3' | 57.7 | 41 |
| | 5'-aaaaatgtacttcagggcttggtcaata-3' | 56.9 | 42 |
| | 5'-aaaatgtacttcagggcttggtcaata-3' | 56.5 | 43 |
| | 5'-aaatgtacttcagggcttggtcaata-3' | 56.2 | 44 |
| | 5'-aatgtacttcagggcttggtcaata-3' | 55.8 | 45 |
| | 5'-atgtacttcagggcttggtcaata-3' | 55.4 | 46 |
| | 5'-tgtacttcagggcttggtcaata-3' | 55.1 | 47 |
| | 5'-gtacttcagggcttggtcaata-3' | 53.6 | 48 |
| | 5'-tacttcagggcttggtcaata-3' | 52.3 | 49 |
| | 5"acttcagggcttggtcaata-3' | 52.4 | 50 |
| | 5'-cttcagggcttggtcaata-3' | 50.7 | 51 |
| | 5'-ttcagggcttggtcaata-3' | 49.2 | 52 |

Furthermore, each primer of the aforementioned primer sets (1) and (2) may be, for example, one with the 5' end to which any conventionally known sequence has been added in order to increase the amplification reaction temperature.

Preferably, a primer set for amplifying the CYP2C19 gene of the present invention including at least one of the aforementioned primer sets (1) and (2) is used, for example, in amplifying the CYP2C19 gene in a biological sample such as a whole blood sample. Particularly, when the primer set for amplifying the CYP2C19 gene of the present invention is used in combination with a probe for detecting a polymorphism as described later, it is preferable that the ratio of the whole blood sample to be added to the reaction solution for amplifying a gene be 0.1 to 0.5 vol%. This will be described later.

### < Reagent for amplifying CYP2C19 gene>

As described above, a reagent for amplifying the CYP2C19 gene of the present invention is a reagent for amplifying the CYP2C19 gene by a gene amplification method, wherein the reagent includes a primer set for amplifying the CYP2C19 gene of the present invention. The reagent for amplifying the CYP2C19 gene of the present invention is characterized by including a primer set of the present invention and, for example, components other than this are not limited by any means.

For example, in order to detect an amplification product obtained by a gene amplification method in which a primer set of the present invention is used, the reagent for amplifying the CYP2C19 gene of the present invention further may include a probe that can hybridize to a site to be detected in the CYP2C19 gene. As described above, the primer set of the present invention allows amplification products of one or two target regions in the CYP2C19 gene to be obtained by a gene amplification method according to, for example, the type of the primer sets (1) and (2) included therein. Accordingly, when a probe complementary to the sequence to be detected in each target region described above is allowed to coexist, for example, the presence or absence of amplification or the genotype (polymorphism) of the site to be detected can be detected by the method described later. Such probes and the method of using them are explained later in the description of the polymorphism analysis method. Furthermore, it is preferable that the reagent for amplifying the CYP2C19 gene of the present invention be used in amplifying the CYP2C19 gene in a biological sample such as whole blood. Particularly, when the reagent for amplifying the CYP2C19 gene of the present invention is used in combination with the probe described above, it is preferable that the ratio of the whole blood sample to be added to the reaction solution for amplifying a gene be 0.1 to 0.5 vol%. In the present invention, the term "sequence to be detected" denotes a sequence including a site (site to be detected) at which a polymorphism is generated.

The form of the reagent for amplifying the CYP2C19 gene of the present invention is not particularly limited and it may be, for example, a liquid reagent containing a primer set for amplifying the CYP2C19 gene of the present invention or a dry reagent that is to be suspended in a solvent before use. Furthermore, the content of the primer set for amplifying the CYP2C19 gene also is not particularly limited.

### <Method of manufacturing amplification product>

As described above, the method of manufacturing an amplification product of the present invention is a method of manufacturing an amplification product of the CYP2C19 gene by a gene amplification method, wherein the method includes the following step (1):
(I) amplifying the CYP2C19 gene in a reaction solution using a primer set for amplifying the CYP2C19 gene of the present invention, with nucleic acid contained in a sample being used as a template.

When a primer set of the present invention is used to perform an amplification reaction in this manner, the target region of the CYP2C19 gene can be amplified as described above. Furthermore, when the primer set for amplifying the CYP2C19 gene of the present invention includes both of the primer sets (1) and (2), two target regions including two sites to be detected, respectively, in CYP2C19 can be amplified simultaneously in the same reaction solution. The target regions to be amplified according to the present invention are regions including the sites to be detected at which respective polymorphisms (CYP2C19*2 and CYP2C19*3) are generated, respectively, as described above. The method of manufacturing an amplification product of the present invention is characterized in that a primer set of the present invention is used, and, for example, the type of and conditions for the gene amplification method are not limited by any means.

The gene amplification method is not particularly limited as described above. Examples thereof include the polymerase chain reaction (PCR) method, a nucleic acid sequence based amplification (NASBA) method, a transcription-mediated amplification (TMA) method, and a strand displacement amplification (SDA) method. Particularly, the PCR method is preferable. The present invention is described below using the PCR method as an example but is not limited thereby.

The sample to which the present invention is to be applied is not particularly limited as long as it contains, for example, nucleic acid to serve as a template. However, it is preferable that the present invention be applied to, for example, a contaminated sample. Examples of the contaminated sample include whole blood, cells in the mouth (for example, oral mucosa), somatic cells of nails and hairs, germ cells, expectoration, amniotic fluid, paraffin-embedded tissue, urine, gastric juice (for example, gastric lavage fluid), and suspensions thereof. According to the method of manufacturing an amplification product using a primer set of the present invention, for example, even in the case of a sample (particularly, a biological sample such as whole blood or cells in the mouth) with various contaminants, the method is less subject to the effect thereof and allows the target region in the CYP2C19 gene to be amplified specifically. Thus, according to the present invention, even a highly contaminated sample, which is difficult to use in the conventional methods, can be used as it is, for instance, without being pretreated, for example, without being purified. Therefore, it can be said that an amplification product can be prepared quicker as compared to the conventional method also from the viewpoint of the pretreatment of the sample.

The ratio of the sample to be added to the reaction solution is not particularly limited. Specifically, when the sample is a biological sample (for example, a whole blood sample), the lower limit of the ratio thereof to be added to the reaction solution is, for example, preferably at least 0.01 vol%, more preferably at least 0.05 vol%, and further preferably at least 0.1 vol%. Furthermore, the upper limit of the ratio thereof to be added also is not particularly limited and is, for example, preferably 2 vol% or lower, more preferably 1 vol% or lower, and further preferably 0.5 vol% or lower.

When an optical detection to be described later is intended to be performed, particularly, when an optical detection is performed using a labeled probe, it is preferable that the ratio of a biological sample, such as a whole blood sample, to be added be set at, for example, 0.1 to 0.5 vol%. Generally, in the PCR reaction, a heat treatment is carried out to denature DNA (i.e. to dissociate it into a single-stranded DNA). This heat treatment may denature, for example, sugar or protein contained in the sample and thereby may generate an insolubilized precipitate or turbidity. Therefore, when the presence or absence of an amplification product or the genotype (polymorphism) of a site to be detected is to be checked by an optical method, the generation of such a precipitate or turbidity may affect measurement accuracy. However, when the ratio of the whole blood sample to be added to the reaction solution is set in the range described above, for example, an effect caused by generation of, for example, a precipitate due to denaturation can be prevented sufficiently and thereby the accuracy of measurement carried out by the optical method can be improved, although the mechanism thereof is unknown. Furthermore, since it also sufficiently can prevent PCR from being inhibited due to the contaminants contained in a whole blood sample, the amplification efficiency can be improved further. Accordingly, when in addition to the use of a primer set of the present invention, the ratio of the sample such as a whole blood sample to be added is set in the aforementioned range, the need to pretreat the sample further can be omitted.

Furthermore, the ratio of the whole blood sample in the reaction solution can be indicated not by the aforementioned volume ratio (for example, 0.1 to 0.5 vol%) but by a weight ratio of hemoglobin (hereinafter referred to as "Hb"). In this case, the ratio of the whole blood sample in the reaction solution is, for example, preferably in the range of 0.565 to 113 g/L, more preferably in the range of 2.825 to 56.5 g/L, and further preferably in the range of 5.65 to 28.25 g/L, in terms of the amount of Hb. The ratio of the whole blood sample to be added during the reaction may satisfy, for example, both the volume ratio and the Hb weight ratio, or one of them.

The whole blood may be any one of, for example, hemolyzed whole blood, unhemolyzed whole blood, anticoagulated whole blood, and whole blood containing coagulated fractions.

In the present invention, the target nucleic acid contained in a sample is, for example, DNA. The aforementioned DNA may be DNA contained originally in the sample, such as a biological sample, or an amplification product DNA obtained through amplification by a gene amplification method. In the latter case, an example thereof is cDNA that is generated from RNA (for example, total RNA or mRNA) contained originally in the sample by a reverse transcription reaction (for instance, reverse transcription PCR (RT-PCR)).

In the method of manufacturing an amplification product of the present invention, it is preferable that albumin further be added to the reaction solution before the start of a gene amplification reaction. Such addition of albumin further can reduce the effect of generating a precipitate or turbidity described above and also further can improve the amplification efficiency. Specifically, it is preferable that albumin be added before the amplification reaction in step (I) or a step of dissociation into a single-stranded DNA.

The ratio of albumin to be added to the reaction solution is, for example, in the range of 0.01 to 2 wt%, preferably 0.1 to 1 wt%, and more preferably 0.2 to 0.8 wt%. The albumin is not particularly limited. Examples thereof include bovine serum albumin (BSA), human serum albumin, rat serum albumin, and horse serum albumin. One of them may be used or two or more of them may be used in combination.

Next, a method of manufacturing an amplification product of the present invention is described using an example in which, with respect to a whole blood sample including DNA as target nucleic acid, amplification products of two target regions of the CYP2C19 gene are produced by PCR using primer sets for amplifying the CYP2C19 gene of the present invention including both of the aforementioned primer sets (1) and (2). The present invention is characterized by using primer sets of the present invention and other configurations and conditions are not limited by any means.

First, a PCR reaction solution is prepared. The ratio of the primer sets of the present invention to be added is not particularly limited. However, it is preferable that F primers of the primer sets (1) and (2) each be added to be 0.1 to 2 µmol/L, more preferably 0.25 to 1.5 µmol/L, and particularly preferably 0.5 to 1 µmol/L. Furthermore, it is preferable that R primers of the primer sets (1) and (2) each be added to be 0.1 to 2 µmol/L, more preferably 0.25 to 1.5 µmol/L, and particularly preferably 0.5 to 1 µmol/L. The ratio (F:R, molar ratio) between the F primer and the R primer to be added to each primer set is not particularly limited. It is, for example, preferably 1 : 0.25 to 1: 4 and more preferably 1: 0.5 to 1: 2.

The ratio of the whole blood sample in the reaction solution is not particularly limited but is preferably in the range described above. The whole blood sample may be added to the reaction solution without being treated or may be added to the reaction solution after being diluted with a solvent such as water or a buffer solution beforehand. When the whole blood sample is diluted beforehand, the dilution ratio is not particularly limited. It can be set so that, for example, the final ratio of the whole blood added to the reaction solution is in the aforementioned range, for example, 1:100 to 1:2000 and preferably 1:200 to 1:1000.

Other composition components in the reaction solution are not particularly limited and can be conventionally known components, whose ratios also are not particularly limited. Examples of the composition components include DNA polymerase, nucleotide (nucleoside triphosphate (dNTP)), and a solvent. Furthermore, as described above, it is preferable that the reaction solution further contain albumin. In the reaction solution, the order of addition of the respective composition components is not limited by any means.

The DNA polymerase is not particularly limited and, for example, a conventionally known thermoduric bacteria-derived polymerase can be used. Specifically, for example, *Thermus aquaticus*-derived DNA polymerase (USP 4,889,818 and USP 5,079,352) (trade name: Taq polymerase), *Thermus thermophilus-derived* DNA polymerase (WO 91/09950) (rTth DNA polymerase), *Pyrococcus furiosus-*derived DNA polymerase (WO 92/9689) (Pfu DNA polymerase; manufactured by Stratagenes), and *Thermococcus litoralis*-derived DNA polymerase (EP-A 455 430) (Trademark: Vent; manufactured by New England Biolabs) are commercially available. Particularly, *Thermus aquaticus-*derived thermostable DNA polymerase is preferable.

The ratio of DNA polymerase to be added to the reaction solution is not particularly limited and is, for example, 5 to 50 U/mL, preferably 1 to 100 U/mL, and more preferably 20 to 30 U/mL. With respect to the unit of activity (U) of DNA polymerase, generally, 1 U denotes the activity that allows all 10 nmol of nucleotide to be taken into an acid-insoluble precipitate in 30 minutes at 74°C in a reaction solution for activity measurement, with an activated salmon sperm DNA being used as a template primer. The composition of the reaction solution for activity measurement is, for example, 25 mM TAPS buffer (pH 9.3, 25°C), 50 mM KCl, 2 mM MgCl₂, 1 mM mercaptoethanol, 200 µM dATP, 200 µM dGTP, 200 µM dTTP, 100 µM [α⁻³²P] dCTP, and 0.25 mg/mL activated salmon sperm DNA.

Generally, examples of the nucleoside triphosphate include dNTP (dATP, dCTP, dTTP). The ratio of dNTP to be added to the reaction solution is not particularly limited and is, for example, 0.01 to 1 mmol/L, preferably 0.05 to 0.5 mmol/L, and more preferably 0.1 to 0.3 mmol/L.

Examples of the solvent include buffer solutions such as Tris-HCl, Tricine, MES, MOPS, HEPES, and CAPS. Commercially available PCR buffer solutions or buffer solutions of commercially available PCR kits can be used.

Furthermore, the PCR reaction solution further may contain heparin, betaine, KCl, MgCl₂, MgSO₄, glycerol, etc. The ratios thereof to be added can be set in ranges in which the PCR reaction is not inhibited.

The total volume of the reaction solution is not particularly limited and can be determined suitably according to, for example, the equipment (thermal cycler) to be used. It is generally 1 to 500 µL and preferably 10 to 100 µL.

Subsequently, PCR is performed. The cycle conditions in PCR are not particularly limited, and, for example, (1) dissociation of whole blood-derived double-stranded DNA into single-stranded DNA, (2) annealing of a primer, and (3) elongation of a primer (polymerase reaction) are as described below. Furthermore, the number of cycles also is not particularly limited but preferably is at least 30, with the following three steps (1) to (3) being considered as one cycle. The upper limit thereof, in total, is not particularly limited and is, for example, 100 cycles or less, preferably 70 cycles or less, and further preferably 50 cycles or less. The change in temperature in each step can be controlled automatically using, for example, a thermal cycler. When primer sets of the present invention are used, since they are excellent in amplification efficiency as described above, 50 cycles can be completed in approximately one hour (preferably within one hour) according to the present invention, while it takes approximately three hours to complete 50 cycles according to the conventional methods.

**[Table 3]**

| | | Temperature (°C) and Time (sec) |
|---|---|---|
| (1) | Dissociation of single-stranded DNA | For example, 90 to 99°C, 1 to 120 sec |
| | | Preferably, 92 to 95°C, 1 to 60 sec |
| (2) | Annealing of primer | For example, 40 to 70°C, 1 to 300 sec |
| | | Preferably, 50 to 70°C, 5 to 60 sec |
| (3) | Elongation reaction | For example, 50 to 80°C, 1 to 300 sec |
| | | Preferably, 50 to 75°C, 5 to 60 sec |

In the manner described above, amplification products complementary to the two target regions in the CYP2C19 gene can be produced. When an amplification product complementary to one of the two target regions is to be produced, a primer set for amplifying the CYP2C19 gene of the present invention containing one of the primer sets (1) and (2) corresponding to the target region(s) can be used.

The method of manufacturing an amplification product of the present invention further may include a step of detecting an amplification product of a target region obtained by the aforementioned amplification reaction. This makes it possible to detect the presence or absence of the amplification product or the genotype (polymorphism, CYP2C19*2 or CYP2C19*3) in the target region in the CYP2C19 gene. The presence or absence of the amplification product can be checked by a conventionally known method. Specifically, it can be checked by, for example, further adding a probe (for instance, a fluorescently-labeled probe) that can hybridize to a site to be detected in the CYP2C19 gene to the reaction solution in step (I), and further in step (II), measuring the fluorescence intensity of the fluorescent label in the probe with respect to the reaction solution. Furthermore, when two target regions are to be amplified, it can be checked by, for example, further adding two probes (for instance, fluorescently-labeled probes) that can hybridize to the respective sites to be detected in the CYP2C19 gene to the reaction solution in step (I), and further in step (II), measuring the fluorescence intensity of the fluorescent label in each probe with respect to the reaction solution. Detection of polymorphisms, CYP2C19*2 and CYP2C19*3, in the CYP2C19 gene is described below as an embodiment of the present invention.

### < CYP2C19 gene polymorphism analysis method>

A CYP2C19 gene polymorphism analysis method of the present invention is a method of analyzing the polymorphism of a site to be detected in the CYP2C19 gene, wherein the method includes the following steps (i) to (iv) :
(i) amplifying a region including a site to be detected in the CYP2C19 gene in a reaction solution by a method of manufacturing an amplification product according to the present invention,
(ii) preparing a reaction solution that contains the amplification product obtained in step (i) and a probe capable of hybridizing to the site to be detected,
(iii) measuring signal values that indicate melting states of a hybridization product between the amplification product and the probe while changing the temperature of the reaction solution, and
(iv) determining a polymorphism of the site to be detected from a change in the signal values accompanying a change in the temperature.

In this manner, when an amplification product is produced using a primer set of the present invention, it is possible to amplify the target region including a site to be detected of a polymorphism (CYP2C19*2 or CYP2C19*3) in the CYP2C19 gene as described above and to analyze the polymorphism of the site to be detected in the target region.

The probe to be used in step (ii) is not particularly limited. Examples thereof include a probe that hybridizes to the site where the polymorphism CYP2C19*2 is generated (hereinafter, also referred to as a "probe for CYP2C19*2"), and a probe that hybridizes to the site where the polymorphism CYP2C19*3 is generated (hereinafter, also referred to as a "probe for CYP2C19*3"). Preferably, these probes each are a probe complementary to a sequence to be detected containing the aforementioned site to be detected. Any one of those probes may be used or both of them may be used. This can be determined, for example, according to the type of the target region(s) amplified with a primer set for amplifying the CYP2C19 gene of the present invention. When two probes are used, for example, the polymorphisms of two sites to be detected can be analyzed using the same reaction solution.

The probes for detecting the polymorphisms are not particularly limited and can be configured by a conventionally known method. For instance, they each may be designed as a sequence to be detected containing a site to be detected of a polymorphism, based on the sequence of a sense strand or the sequence of an antisense strand of the CYP2C19 gene. Furthermore, the base located at the site to be detected of a polymorphism can be determined suitably according to the type of each polymorphism. In other words, in the case of CYP2C19*2, since the polymorphisms of "G" and "A" at base 1361 in SEQ ID NO: 1 have been known, examples of the probe include a probe complementary to either a sequence to be detected including G at base 1361 or a sequence to be detected including A at base 1361 (a probe for detecting a sense strand), and a probe complementary to a sequence of an antisense strand thereof (a probe for detecting an antisense strand). Furthermore, in the case of CYP2C19*3, since the polymorphisms of "G" and "A" at base 155 in SEQ ID NO: 1 have been known, examples of the probe include a probe complementary to either a sequence to be detected including G at base 155 or a sequence to be detected including A at base 155 (a probe for detecting a sense strand), and a probe complementary to a sequence of an antisense strand thereof (a probe for detecting an antisense strand). As described above, when a probe is designed, with the base located at the site to be detected where a polymorphism is generated being set to be any one of the bases as described above, it is also possible to judge what type of polymorphism is expressed at each site to be detected in a CYP2C19 gene by the method as described later.

The probe can be added to an amplified reaction solution after step (i) i.e. after a target region in the CYP2C19 gene is subjected to an amplification reaction. However, it is preferable that the probe be added to a reaction solution before the amplification reaction in step (i) since this allows analysis to be performed easily and quickly.

The ratio of the probe to be added to the reaction solution is not particularly limited. For example, each probe is added to be preferably in the range of 10 to 400 nmol and more preferably in the range of 20 to 200 nmol. When a fluorescent dye is used as the label for a probe, an unlabeled probe with a sequence identical to that of the labeled probe may be used in combination with the labeled probe, for example, in order to adjust the fluorescence intensity to be detected, and the unlabeled probe may include a phosphate group added to the 3' end thereof. In this case, the molar ratio between the labeled probe and the unlabeled probe is preferably, for example, 1: 10 to 10: 1. With respect to the probe for CYP2C19*2 and the probe for CYP2C19*3, it is not particularly limited to which the unlabeled probe is added in combination with the labeled probe. For example, it is preferable that the labeled probe and the unlabeled probe are added to the probe for CYP2C19*3. The length of the probe is not particularly limited. It is, for example, 5- to 50-mers and preferably 10- to 30-mers.

The Tm value is described. When a solution containing double-stranded DNA is heated, the absorbance at 260 nm increases. This is because heating releases the hydrogen bonds between both strands in the double-stranded DNA to dissociate it into single-stranded DNA (i.e. DNA melting). When all double-stranded DNAs are dissociated into single-stranded DNAs, the absorbance thereof indicates approximately 1.5 times that obtained at the start of heating (i.e. absorbance of only double-stranded DNAs), which makes it possible to judge that melting is completed thereby. Based on this phenomenon, the melting temperature Tm generally is defined as a temperature at which the absorbance has reached 50% of the total increase in absorbance.

In the aforementioned step (iii), the measurement of the signal values that indicate the melting states of the hybridization product between the amplification product and the probe may be a measurement of absorbance at 260 nm as described above but may be a measurement of the signal of a labeling substance. Specifically, it is preferable that a labeled probe labeled with a labeling substance be used as the aforementioned probe to perform the measurement of the signal of the labeling substance. The labeled probe can be, for example, a labeled probe that exhibits a signal independently but does not exhibit a signal after hybridization, or a labeled probe that does not exhibit a signal independently but exhibits a signal after hybridization. The former probe does not exhibit a signal after forming a hybrid (double-stranded DNA) with a sequence to be detected but exhibits a signal when the probe is released by heating. On the other hand, the latter probe exhibits a signal after forming a hybrid (double-stranded DNA) with a sequence to be detected but the signal is reduced (quenched) when the probe is released by heating. Accordingly, when the signal exhibited by the label is detected under a condition (absorption wavelength etc.) specific to the signal, the progress of melting of the hybridization product and the Tm value can be determined as in the case of the measurement of absorbance at 260 nm.

In the present invention, as described above, it is also possible to check polymorphisms with respect to amplification products of two target regions amplified in the same reaction solution. Accordingly, when two types of probes are used, it is preferable that they be labeled with different labels, each of which is detected under its own condition. The use of different labels as described above makes it possible to analyze each amplification product separately by changing the detection conditions even in the same reaction solution.

Specific examples of labeling substances in the labeled probes include a fluorescent dye (fluorophore). A specific example of the labeled probes is preferably a probe that, for example, has been labeled with a fluorescent dye, exhibits fluorescence independently, and allows fluorescence to be reduced (for example, quenched) after hybridization. Generally, a probe that utilizes such a fluorescence quenching phenomenon is referred to as a fluorescence quenching probe. Particularly, with respect to the aforementioned probe, it is preferable that the 3' end or 5' end of oligonucleotide be labeled with a fluorescent dye and the base located at the end to be labeled be C. In this case, in the sequence to be detected, to which the labeled probe hybridizes, it is preferable that the base sequence of the labeled probe be designed so that the base to be paired with the end base C of the labeled probe or the base located 1 to 3 bases apart from the base to be paired be G. Generally, such a probe is referred to as a guanine quenching probe and is known as so-called QProbe (registered trademark). When such a guanine quenching probe hybridizes to a sequence to be detected, C located at the end, which has been labeled with a fluorescent dye, approaches G in the DNA to be detected, and thereby a phenomenon occurs in which the emission of the fluorescent dye decreases (the fluorescence intensity decreases). The use of such a probe makes it possible to verify hybridization and dissociation easily according to a change in the signal.

The fluorescent dye is not particularly limited. Examples thereof include fluorescein, phosphor, rhodamine, and polymethine dye derivative. Examples of commercially available fluorescent dye include BODIPY FL (brand name, manufactured by Molecular Probe Inc.), FluorePrime (trade name, manufactured by Amersham Pharmacia), Fluoredite (trade name, manufactured by Millipore Corporation), FAM (manufactured by ABI), Cy3 and Cy5 (manufactured by Amersham Pharmacia), and TAMRA (manufactured by Molecular Probe Inc.). The combination of fluorescent dyes to be used for two types of probes is not particularly limited as long as, for example, it allows the respective probes to be detected under different conditions. Examples thereof include a combination of Pacific Blue (with a detection wavelength of 450 to 480 nm) and BODIPY FL (with a detection wavelength of 515 to 555 nm).

Specific examples of the sequences of probes for detecting the polymorphisms, CYP2C19*2 and CYP2C19*3, are indicated below, but the present invention is not limited thereto. The following probe (1) is an example of the probe for CYP2C19*2 and is a probe for detecting an antisense strand. The following probe (2) is an example of the probe for CYP2C19*3 and is a probe for detecting an antisense strand.

### Probe (1)

At least one oligonucleotide having a sequence identical to that of a region extending from cytosine (C) at base 1373 to be considered as the first base to any one of the 16^{th} to 28^{th} bases in the direction toward the 5' end in SEQ ID NO: 1, with the cytosine being the 3' end.

### Probe (2)

At least one oligonucleotide selected from:
at least one oligonucleotide having a sequence identical to that of a region extending from cytosine (C) at base 146 to be considered as the first base to any one of the 18^{th} to 25^{th} bases in the direction toward the 3' end in SEQ ID NO: 1, with the cytosine being the 5' end, and
at least one oligonucleotide having a sequence identical to that of a region extending from cytosine (C) at base 150 to be considered as the first base to any one of the 14^{th} to 19^{th} bases in the direction toward the 3' end in SEQ ID NO: 1, with the cytosine being the 5' end.

In the probe (1), base 1361 in SEQ ID NO: 1 is A or G. In the probe (2), base 155 in SEQ ID NO: 1 is A or G.

Specific examples of Probe (1) and Probe (2) are indicated in the following table. "Tm(°C)" indicated below in the table is Tm(°C) obtained when each sequence indicated below in the table was hybridized with the sequence perfectly complementary thereto. The "Tm(°C)" is a value calculated by using MELTCALC software (http://www.meltcalc.com/), with parameters including an oligonucleotide concentration of 0.2 µM and a sodium equivalent (Na eq.) of 50 mM.

**[Table 4]**

| Probe | Sequence | | Tm(°C) | SEQ ID NO. |
|---|---|---|---|---|
| Probe (1) for CYP2C19*2 | 5'-cattgattatttcccAggaacccataac-3' | | 56 | 53 |
| | 5'-attgattatttcccAggaacccataac-3' | | 55.1 | 54 |
| | 5'-ttgattatttcccAggaacccataac-3' | | 54.8 | 55 |
| | 5'-tgattatttcccAggaacccataac-3' | | 54.4 | 56 |
| | 5'-gattattteecAggaacccataac-3' | | 53 | 57 |
| | 5"attatttcccAggaacccataac-3' | | 52 | 58 |
| | 5'-ttatttcccAggaacccataac-3' | | 51.6 | 59 |
| | 5'-tatttcccAggaacccataac-3' | | 50.9 | 60 |
| | 5'-atttcccAggaacccataac-3' | | 51 | 61 |
| | 5'-tttcccAggaacccataac-3' | | 50.5 | 62 |
| | 5'-ttcccAggaacccataac-3' | | 49.6 | 63 |
| | 5'-tcccAggaacccataac-3' | | 48.6 | 64 |
| | 5'-cccAggaacccataac-3' | | 46.7 | 65 |
| Probe (2) for CYP2C19*3 | 5'-caccccctgAatccaggtaaggcca-3' | | 63.3 | 66 |
| | 5'-caccccctgAatccaggtaaggcc-3' | | 62.2 | 67 |
| | 5'-caccccctgAatccaggtaaggc-3' | | 60.4 | 68 |
| | 5'-caccccctgAatccaggtaagg-3' | | 57.9 | 69 |
| | 5'-caccccctgAatccaggtaag-3' | | 55.7 | 70 |
| | 5'-caccccctgAatccaggtaa-3' | | 54.7 | 71 |
| | 5'-caccccctgAatccaggta-3' | | 54.1 | 72 |
| | 5'-caccccctgAatccaggt-3' | | 54.4 | 73 |
| | | 5'-ccctgAatccaggtaaggc-3' | 53.4 | 74 |
| | | 5'-ccctgAatccaggtaagg-3' | 49.9 | 75 |
| | | 5'-ccctgAatccaggtaag-3' | 46.8 | 76 |
| | | 5'-ccctgAatccaggtaa-3' | 44.9 | 77 |
| | | 5'-ccctgAatccaggta-3' | 43.4 | 78 |
| | | 5'-ccctgAatccaggt-3' | 43.1 | 79 |

Each probe (1) indicated in the above table consists of a sequence identical to that of a region having A at base 1361 in SEQ ID NO: 1, and the capitalized base indicates the base complementary to base 1361 in SEQ ID NO: 1. In each probe (1), the capitalized base can be replaced by "r", and the "r" may be either A or G. Each probe (2) indicated in the above table consists of a sequence identical to that of a region having A at base 155 in SEQ ID NO: 1, and the capitalized base indicates base 155 in SEQ ID NO: 1. In each probe (2), the capitalized base can be replaced by "r", and the "r" may be either G or A. As described above, specific examples of the probe according to the present invention may be strands complementary to oligonucleotides indicated in the above table.

The aforementioned probes are examples and the present invention is not limited thereto. With respect to the probe for CYP2C19*2, a preferable probe is (P1') oligonucleotide consisting of the base sequence of SEQ ID NO: 61 or SEQ ID NO: 64. With respect to the probe for CYP2C19*3, a preferable probe is (P2') oligonucleotide consisting of the base sequence of SEQ ID NO: 69 or SEQ ID NO: 76.

For example, when two types of probes are used, as described above, it is preferable that they be labeled with different fluorescent dyes (fluorescent dyes that are detected at different wavelengths). For instance, when the probes indicated in the above table are guanine quenching probes, it is preferable that in each probe for CYP2C19*2 (P1 probe), cytosine at the 3' end thereof be labeled with a fluorescent dye (for instance, Pacific Blue or TAMRA) as described above and in each probe for CYP2C19*3 (P2 probe), cytosine at the 5' end thereof be labeled with a fluorescent dye (for instance, BODIPY FL) as described above. Furthermore, a probe with the 5' end labeled with a fluorescent dye may have the 3' end, to which a phosphate group further may be added, in order to prevent the probe itself from elongating.

Next, with respect to the detection method of the present invention, a method of detecting two polymorphisms, CYP2C19*2 and CYP2C19*3, in the CYP2C19 gene using the following probes is described as an example. However, the present invention is not limited thereto.

### <Probes>

Probe for CYP2C19*2
   5'-atttcccAggaacccataac-(Pacific Blue)-3' (SEQ ID NO: 61), or
   5'-tcccaggaacccataac-(BODIPY FL)-3' (SEQ ID NO: 64)
Probe for CYP2C19*3
   5'-(BODIPY FL)-caccccctgAatccaggtaagg-P-3' (SEQ ID NO: 69), or
   5'-(BODIPY FL)-ccctgAatccaggtaag-P-3' (SEQ ID NO: 76)

First, using a reaction solution containing the aforementioned two labeled probes added thereto, PCR was performed as described above, and thereby the two regions of the CYP2C19 gene are amplified at the same time in the same reaction solution. The reaction solution contains, for example, a primer set for amplifying the CYP2C19 gene of the present invention, DNA polymerase, dNTP, a sample containing nucleic acid to serve as a template, and the aforementioned two probes. In addition to them, various additives that can be used for amplifying nucleic acid may be contained.

Next, the amplification products thus obtained are dissociated and then single-stranded DNA obtained through dissociation is hybridized with the labeled probes. This can be carried out through, for example, a change in the temperature of the reaction solution.

The heating temperature in the dissociation step is not particularly limited as long as it allows the amplification products to be dissociated. It is, for example, 85 to 95°C. The heating time also is not particularly limited and generally is 1 second to 10 minutes and preferably 1 second to 5 minutes.

The dissociated single-stranded DNAs can be hybridized with the labeled probes by, for example, decreasing the heating temperature employed in the dissociation step after the dissociation step. The temperature condition is, for example, 40 to 50°C.

The temperature of the reaction solution is changed and thereby signal values that indicate the melting states of hybridization products between the amplification products and the labeled probes are measured. Specifically, for example, the reaction solution (the hybridization products between the single-stranded DNAs and the labeled probes) is heated, and thereby the change in the signal values accompanying the temperature rise is measured. As described above, when, for example, a probe (guanine quenching probe), in which the base C at the end has been labeled, is used, fluorescence decreases (or quenches) in the state where the probe has been hybridized with the single-stranded DNA, while fluorescence is emitted in the state where the probe has been dissociated. Accordingly, for example, the hybridization product in which the fluorescence has decreased (or quenched) is heated gradually and the increase in fluorescence intensity accompanying the temperature rise may be measured.

The temperature range in which the change in fluorescence intensity is to be measured is not particularly limited. For example, the start temperature is room temperature to 85°C and preferably 25 to 70°C, while the end temperature is, for example, 40 to 105°C. Furthermore, the rate of temperature rise is not particularly limited and is, for example, 0.1 to 20°C/sec and preferably 0.3 to 5°C/sec.

Next, the Tm value is determined by analyzing a change in the signal. Specifically, the amount of change in the fluorescence intensity per unit time at each temperature (-d fluorescence intensity increase/dt) is calculated from the fluorescence intensity obtained and the temperature at which the lowest value is obtained is determined as the Tm value. It is also possible to determine, as the Tm value, the point at which the amount of increase in the fluorescence intensity per unit time (fluorescence intensity increase/t) is the highest. On the contrary, the amount of decrease in the fluorescence intensity is measured when the labeled probe used is not a quenching probe but a probe that does not exhibit a signal independently but exhibits a signal after hybridization.

In the present invention, in order to detect two polymorphisms, CYP2C19*2 and CYP2C19*3, the respective Tm values are determined under conditions according to the respective labels of the two probes. Pacific Blue, a probe for CYP2C19*2, can be detected with, for example, a detection wavelength of 450 to 480 nm, and BODIPY FL, a probe for CYP2C19*3, can be detected with, for example, a detection wavelength of 515 to 555 nm. Further, when the label is TAMRA, it can be detected with, for example, a detection wavelength of 585 to 700 nm.

From such Tm values, the genotypes in the respective sites to be detected are determined. In the Tm analysis, the case of a perfectly complementary hybrid (match) results in a higher Tm value indicating dissociation than that obtained in the case of a hybrid including a different single base (mismatch). Accordingly, when with respect to the probe, the Tm value obtained in the case of a perfectly complementary hybrid and the Tm value obtained in the case of a hybrid including a different single base are determined beforehand, the genotype at each site to be detected can be determined. For example, in the case where the base located at the site to be detected is assumed to be of a mutation type (with, for instance, A at base 1361 in SEQ ID NO: 1), when using a probe complementary to the sequence to be detected containing the base, the polymorphism of the amplification product can be judged as a mutation type if the Tm value of the resultant hybrid is equal to the Tm value of a perfectly complementary hybrid. Furthermore, the polymorphism of the amplification product can be judged as a wildtype (with, for example, G at base 1361 in SEQ ID NO: 1) if the Tm value of the resultant hybrid is equal to the Tm value of the hybrid including a different single base (i.e. a lower value than the Tm value of the perfectly complementary hybrid). Moreover, when both the Tm values are detected, it can be judged as heterozygote. Thus, the genotypes of the polymorphisms, CYP2C19*2 and CYP2C19*3, can be judged from the two Tm values with respect to the respective labeled probes.

In the present invention, for example, a change in the signal during hybridization may be measured instead of the method in which the temperature of a reaction solution containing the probes is increased (a hybridization product is heated) and a change in the signal accompanying the temperature rise is measured as described above. In other words, when the temperature of the reaction solution containing the aforementioned probes is decreased to form hybridization products, the change in the signal accompanying the temperature decrease may be measured.

Specifically, when using a labeled probe that exhibits a signal independently but does not exhibit a signal after hybridization (for example, a guanine quenching probe), the labeled probe emits fluorescence in the state where single-stranded DNA and the probe are dissociated, but the fluorescence decreases (or quenches) when a hybrid is formed through temperature decrease. Accordingly, for example, the temperature of the reaction solution is decreased gradually and the decrease in fluorescence intensity accompanying the temperature decrease may be measured. On the other hand, when using a labeled probe that does not exhibit a signal independently but exhibits a signal after hybridization, the labeled probe does not emit fluorescence in the state where single-stranded DNA and the probe are dissociated, but the fluorescence is emitted when a hybrid is formed through temperature decrease. Accordingly, for example, the temperature of the reaction solution is decreased gradually and thereby the increase in fluorescence intensity accompanying the temperature decrease may be measured.

When one of the two types of polymorphisms (CYP2C19*2 and CYP2C19*3) in the CYP2C19 gene are to be analyzed, for instance, a primer set for amplifying the CYP2C19 gene of the present invention may be used that includes one type of primer set corresponding to the target regions that are selected from the primer sets (1) and (2), and furthermore, one type of probe that hybridize to target sites to be detected may be used.

Next, examples of the present invention are described. However, the present invention is not limited by the following examples.

### [Example 1]

Blood was collected from four subjects using heparin lithium blood collection tubes (Samples 1 to 4). Subsequently, 10 µL of blood thus obtained and 90 µL of distilled water were mixed together. Further, 10 µL of this mixture and 90 µL of distilled water were mixed together. Thereafter, 10 µL of the mixture was added to 40 µL of PCR reaction solution having the following composition, and then PCR was performed using a thermal cycler. Conditions for PCR were as follows. That is, after treating at 95°C for 60 seconds, one cycle of treatment at 95°C for 1 second and at 54°C for 10 seconds was repeated for 50 cycles, and further it was treated at 95°C for 1 second and at 40°C for 60 seconds. Subsequently, the PCR reaction solution was heated from 40°C to 95°C at a rate of temperature rise of 1°C/3 seconds, and the change in fluorescence intensity over time was measured. The measurement wavelength was 450 to 480 nm (for detection of the fluorescent dye, Pacific Blue), and 515 to 555 nm (for detection of the fluorescent dye, BODIPY FL). The time required for 50 cycles of PCR was approximately one hour.

**[Table 5]**

| <PCR reaction solution; unit: µl> | |
|---|---|
| Distilled water | 20.875 |
| 5% NaN₃ | 0.5 |
| 20% BSA | 1 |
| 40% Glycerol | 3.125 |
| 10 × Gene Taq buffer * | 5 |
| 2.5 mM dNTPs | 4 |
| 1.00 mM MgCl₂ | 0.75 |
| 5 µM probe for CYP2C19*2 | 1 |
| 5 µM probe 1 for CYP2C19*3 | 1 |
| 5 µM probe 2 for CYP2C19*3 | 1 |
| 100 µM CYP2C19*2 F1 primer | 0.25 |
| 100 µM CYP2C19*2 R1 primer | 0.5 |
| 100 µM CYP2C19*3 F2 primer | 0.25 |
| 100 µM CYP2C19*3 R2 primer | 0.5 |
| 5 U/µl Gene Taq FP * | 0.25 |
| Total | 40 µL |

| | |
|---|---|
| * Trade name, Gene Taq FP: manufactured by Nippon Gene Co., Ltd. | |

### <Probe>

Probe for CYP2C19*2

   5'-atttcccaggaacccataac-(Pacific Blue)-3' (SEQ ID NO: 61)
Probe 1 for CYP2C19*3

   5'-(BODIPY FL)-caccecctgaatecaggtaagg-P-3' (SEQ ID NO: 69)
Probe 2 for CYP2C19*3

   5'-caccccctgaatccaggtaagg-P-3' (SEQ ID NO: 69)

### <Primer set>

CYP2C19*2 F1 primer

   5'-gttttctcttagatatgcaataattttccca-3' (SEQ ID NO: 12)
CYP2C19*2 R1 primer

   5'-cgagggttgttgatgtccatc-3' (SEQ ID NO: 22)
CYP2C19*3 F2 primer

   5'-gaaaaattgaatgaaaacatcaggattgta-3' (SEQ ID NO: 32)
CYP2C19*3 R2 primer

   5'-gtacttcagggcttggtcaata-3' (SEQ ID NO: 48)

The Tm value of a hybrid that matches with the probe for CYP2C19*2 is 60.5°C and that of a hybrid that mismatches therewith is 53.0°C, the Tm value of a hybrid that matches with the probe for CYP2C19*3 is 66.5°C and that of a hybrid that mismatches therewith is 60.0°C.

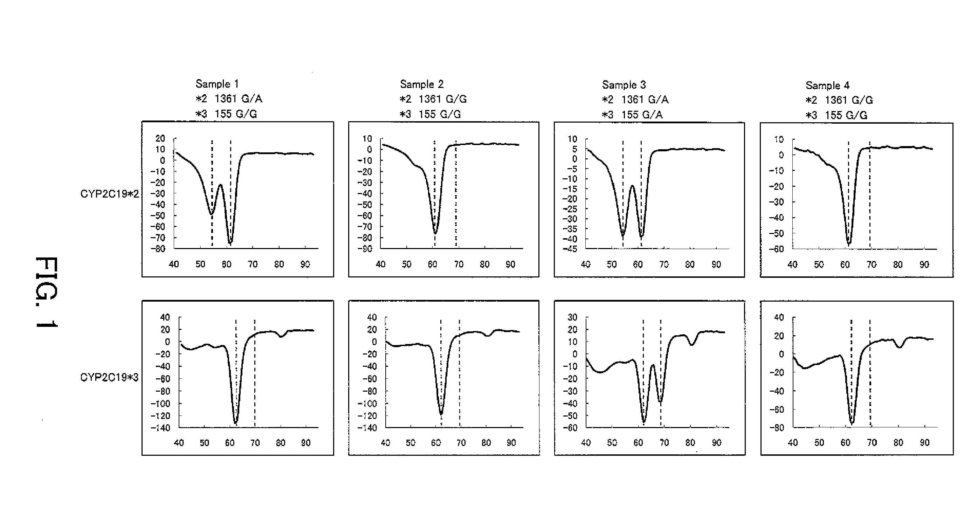

Results of Samples 1 to 4 are indicated in FIG. 1. FIG.1 shows graphs of Tm analysis that indicate the changes in fluorescence intensity accompanying temperature rise. The differential value of the vertical axis indicates "-d fluorescence intensity increase/dt", while the horizontal axis indicates temperature (the same applies below). As shown in these graphs, the genotypes of CYP2C19*2 and CYP2C19*3 in each sample were determined from the peaks of the signals. In order to support the results of these examples, with respect to four subjects, the genotypes of CYP2C19*2 and CYP2C19*3 were confirmed by the RFLP method and the sequencing method. As a result, the same results as those obtained in the example were obtained. Accordingly, the use of a primer set of the present invention made it possible to amplify two regions of the CYP2C19 gene simultaneously in the same reaction solution using a whole blood sample that had not been pretreated and to analyze the two types of polymorphisms using the same reaction solution.

### [Example 2]

Blood was collected from two subjects using EDTA blood collection tubes (Samples 1 and 2). Subsequently, 10 µL of blood thus obtained and 70 µL of diluent A described below were mixed together. Further, 10 µL of this mixture and 70 µL of diluent B described below were mixed together. Subsequently, 10 µL of the mixture thus obtained was heat-treated at 95°C for five minutes. Thereafter, this was added to 46 µL of PCR reaction solution having the following composition, and then PCR was performed using a thermal cycler. Conditions for PCR were as follows. That is, after treating at 95°C for 60 seconds, one cycle of treatment at 95°C for 1 second and at 64°C for 15 seconds was repeated for 50 cycles, and further it was treated at 95°C for 1 second and at 40°C for 60 seconds. Subsequently, the PCR reaction solution was heated from 40°C to 75°C at a rate of temperature rise of 1°C/3 seconds, and the change in fluorescence intensity over time was measured. The measurement wavelength was 515 to 555 nm (for detection of the fluorescent dye, BODIPY FL) and 585 to 700 nm (for detection of the fluorescent dye, TAMRA).

### <Diluent A>

10 mM Tris-HCl (pH 8), 0.1 mM EDTA, 0.05% NaN₃, 0.3% SDS

### <Diluent B>

10 mM Tris-HCl (pH 8), 0.1 mM EDTA, 0.05% NaN₃

**[Table 6]**

| <PCR reaction solution; unit: µl> | |
|---|---|
| Distilled water | 14.25 |
| 5% NaN₃ | 0.5 |
| 20% BSA | 0.5 |
| 40% Glycerol | 12.5 |
| 10 × Gene Taq buffer * | 5 |
| 2.5 mM dNTPs | 4 |
| 100 mM MgCl₂ | 1.5 |
| 5 µM probe for CYP2C19*2 | 4 |
| 5 µM probe for CYP2C19*3 | 2 |
| 100 µM CYP2C19*2 F1 primer | 0.25 |
| 100 µM CYP2C19*2 R1 primer | 0.5 |
| 100 µM CYP2C19*3 F2 primer | 0. 25 |
| 100 µM CYP2C19*3 R2 primer | 0. 5 |
| 5 U/µl Gene Taq FP * | 0.25 |
| Total | 46 µL |

| | |
|---|---|
| * Trade name, Gene Taq FP: manufactured by Nippon Gene Co., Ltd. | |

### <Probes>

Probe for CYP2C19*2

   5'-tcccaggaacccataac-(BODIPY FL)-3' (SEQ ID NO: 64)
Probe for CYP2C19*3

   5'- (TAMRA)-ccctgaatccaggtaag-P-3' (SEQ ID NO: 76)

### <Primer set>

CYP2C19*2 F1 primer

   5'-taaattattgttttctcttagatatgcaataattttccca-3' (SEQ ID NO: 3)

   CYP2C19*2 R1 primer

   5'-cccgagggttgttgatgtccatc-3' (SEQ ID NO: 20)
CYP2C19*3 F2 primer

   5'-tgatggaaaaattgaatgaaaacatcaggattgta-3' (SEQ ID NO: 27)

   CYP2C19*3 R2 primer

   5'-tcaaaaatgtacttcagggcttggtcaata-3' (SEQ ID NO: 40)

The Tm value of a hybrid that matches with the probe for CYP2C19*2 is 59°C and that of a hybrid that mismatches therewith is 51°C, the Tm value of a hybrid that matches with the probe for CYP2C19*3 is 58°C and that of a hybrid that mismatches therewith is 50°C.

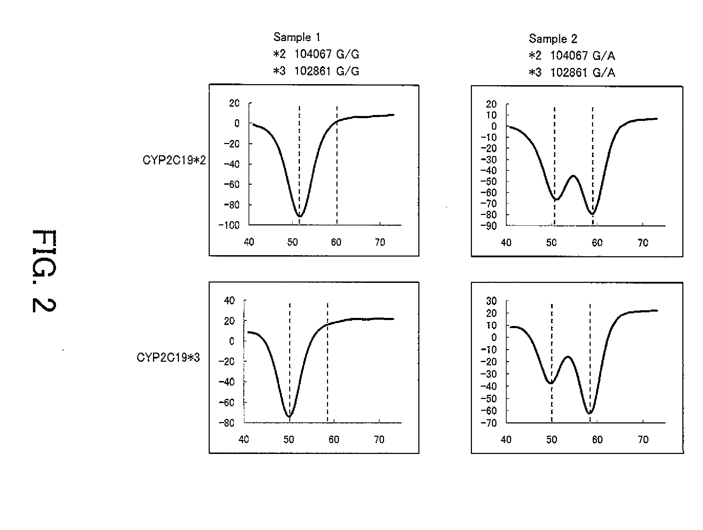

Results of Samples 1 and 2 are indicated in FIG. 2. FIG. 2 shows graphs of Tm analysis that indicate the changes in fluorescence intensity accompanying temperature rise. The differential value of the vertical axis indicates "-d fluorescence intensity increase/dt", while the horizontal axis indicates temperature. As shown in these graphs, the genotypes of CYP2C19*2 and CYP2C19*3 in each sample were determined from the peaks of the signals. In order to support the results of these examples, with respect to two subjects, the genotypes of CYP2C19*2 and CYP2C19*3 were confirmed by the RFLP method and the sequencing method. As a result, the same results as those obtained in the example were obtained. Accordingly, the use of a primer set of the present invention made it possible to amplify two regions of the CYP2C19 gene simultaneously in the same reaction solution using a whole blood sample that had not been pretreated and to analyze the two types of polymorphisms using the same reaction solution.

### Industrial Applicability

As described above, the primer set of the present invention makes it possible to specifically and efficiently amplify a region including a site where a particular polymorphism (CYP2C19*2 or CYP2C19*3) is generated in the CYP2C19 gene. This allows time and cost to be reduced, which is different from the conventional methods as described above. Furthermore, as described above, since the region including a site to be detected of a polymorphism is amplified specifically, for example, the use of a probe complementary to a sequence to be detected including the site to be detected makes it possible to perform Tm analysis directly using the aforementioned reaction solution to type the polymorphism. Moreover, since amplification and typing can be carried out using one reaction solution, the operation can be automated. The use of the primer set of the present invention allows a pretreatment to be omitted even in the case of, for example, a contaminated sample (for instance, whole blood or oral mucosa), and therefore the amplification reaction can be carried out quicker and more easily. Furthermore, when the primer set of the present invention is used, the amplification reaction can be carried out with higher amplification efficiency as compared to conventional cases and thus the reaction time can also be shortened. According to the primer set of the present invention, the reagent including the same, as well as the method of manufacturing an amplification product using them, since the polymorphism in the CYP2C19 gene can be analyzed quickly and simply, it can be said that they are considerably effective in the field of medicine.
## Claims

1. A primer set for amplifying the CYP2C19 gene by a gene amplification method,
wherein the primer set includes at least one of the following primer sets (1) and (2):
a primer set of a pair of primers including a forward primer composed of the following oligonucleotide (F1) and a reverse primer composed of the following oligonucleotide (R1):
(F1): at least one oligonucleotide having a sequence identical to that of a region extending from adenine (A) at base 1341 to be considered as the first base to any one of the 25^{th} to 41^{st} bases in the direction toward the 5' end in the base sequence of SEQ ID NO: 1, with the adenine (A) being the 3' end,
(R1): at least one oligonucleotide complementary to a region extending from guanine (G) at base 1442 to be considered as the first base to any one of the 19^{th} to 24^{th} bases in the direction toward the 3' end in the base sequence of SEQ ID NO: 1, with cytosine (C) complementary to the guanine (G) at base 1442 being the 3' end, and
Primer set (2):
a primer set of a pair of primers including a forward primer composed of the following oligonucleotide (F2) and a reverse primer composed of the following oligonucleotide (R2):
(F2): at least one oligonucleotide having a sequence identical to that of a region extending from adenine (A) at base 143 to be considered as the first base to any one of the 23^{rd} to 37^{th} bases in the direction toward the 5' end in the base sequence of SEQ ID NO: 1, with the adenine (A) being the 3' end,
and
(R2): at least one oligonucleotide complementary to a region extending from thymine (T) at base 231 to be considered as the first base to any one of the 18^{th} to 30^{th} bases in the direction toward the 3' end in the base sequence of SEQ ID NO: 1, with adenine (A) complementary to the thymine (T) at base 231 being the 3' end.

2. The primer set for amplifying the CYP2C19 gene according to claim 1,
wherein the primer sets (1) and (2) are the following primer sets (1') and (2'), respectively:
Primer set (1'):
a primer set of a pair of primers including a forward primer composed of the following oligonucleotide (F1') and a reverse primer composed of the following oligonucleotide (R1'):
(F1'): at least one oligonucleotide selected from oligonucleotide consisting of the base sequence of SEQ ID NO: 3 and oligonucleotide consisting of the base sequence of SEQ ID NO: 12, and
(R1') at least one oligonucleotide selected from oligonucleotide consisting of the base sequence of SEQ ID NO: 20 and oligonucleotide consisting of the base sequence of SEQ ID NO: 22,
Primer set (2'):
a primer set of a pair of primers including a forward primer composed of the following oligonucleotide (F2') and a reverse primer composed of the following oligonucleotide (R2'):
(F2'): at least one oligonucleotide selected from oligonucleotide consisting of the base sequence of SEQ ID NO: 27 and oligonucleotide consisting of the base sequence of SEQ ID NO: 32, and
(R2') at least one oligonucleotide selected from oligonucleotide consisting of the base sequence of SEQ ID NO: 40 and oligonucleotide consisting of the base sequence of SEQ ID NO: 48.

3. The primer set for amplifying the CYP2C19 gene according to claim 1,
wherein the primer set for amplifying the CYP2C19 gene is a primer set for amplifying the CYP2C19 gene in a biological sample.

4. The primer set for amplifying the CYP2C19 gene according to claim 3,
wherein the biological sample is whole blood.

5. A reagent for amplifying the CYP2C19 gene by a gene amplification method,
wherein the reagent comprises a primer set for amplifying the CYP2C19 gene according to claim 1.

6. The reagent for amplifying the CYP2C19 gene according to claim 5, further comprising a probe that can hybridize to a site to be detected in the CYP2C19 gene.

7. The reagent for amplifying the CYP2C19 gene according to claim 6,
wherein the probe is at least one probe of the following oligonucleotides (P1') and (P2'):
(P1') at least one oligonucleotide selected from oligonucleotide consisting of the base sequence of SEQ ID NO: 61 and oligonucleotide consisting of the base sequence of SEQ ID NO: 64,
(P2') at least one oligonucleotide selected from oligonucleotide consisting of the base sequence of SEQ ID NO: 69 and oligonucleotide consisting of the base sequence of SEQ ID NO: 76.

8. The reagent for amplifying the CYP2C19 gene according to claim 6,
wherein the probe is a fluorescently-labeled probe.

9. A method of manufacturing an amplification product of the CYP2C19 gene by a gene amplification method,
wherein the method comprises the following process (I):
(I) amplifying the CYP2C19 gene in a reaction solution using a primer set for amplifying the CYP2C19 gene according to claim 1, with nucleic acid contained in a sample being used as a template.

10. The method of manufacturing an amplification product according to claim 9, wherein a probe that can hybridize to a site to be detected in the CYP2C19 gene further is added to the reaction solution in the process (I).

11. The method of manufacturing an amplification product according to claim 10, wherein the probe is at least one probe of the following oligonucleotides (P1') and (P2'):
(P1') at least one oligonucleotide selected from oligonucleotide consisting of the base sequence of SEQ ID NO: 61 and oligonucleotide consisting of the base sequence of SEQ ID NO: 64,
(P2') at least one oligonucleotide selected from oligonucleotide consisting of the base sequence of SEQ ID NO: 69 and oligonucleotide consisting of the base sequence of SEQ ID NO: 76.

12. The method of manufacturing an amplification product according to claim 10, wherein the probe is a fluorescently-labeled probe.

13. The method of manufacturing an amplification product according to claim 12, wherein the method further comprises the following process (II):
(II) measuring a fluorescence intensity of a fluorescent label contained in the fluorescently-labeled probe in the reaction solution.

14. The method of manufacturing an amplification product according to claim 9, wherein the sample is a biological sample.

15. The method of manufacturing an amplification product according to claim 14, wherein the biological sample is whole blood.

16. The method of manufacturing an amplification product according to claim 15, wherein the ratio of the whole blood sample to be added to the reaction solution is 0.1 to 0.5 vol%.

17. A polymorphism analysis method of analyzing a polymorphism of a site to be detected in the CYP2C19 gene, wherein the method comprises the following processes (i) to (iv):
(i) amplifying a region including a site to be detected in the CYP2C19 gene in a reaction solution by a method of manufacturing an amplification product according to claim 9,
(ii) preparing a reaction solution that contains the amplification product obtained in process (i) and a probe capable of hybridizing to the site to be detected,
(iii) measuring signal values that indicate melting states of a hybridization product between the amplification product and the probe while changing the temperature of the reaction solution, and
(iv) determining a polymorphism of the site to be detected from a change in the signal values accompanying a change in the temperature.

18. The polymorphism analysis method according to claim 17, wherein in the process (i), a probe that can hybridize to the site to be detected is added to the reaction solution prior to an amplification reaction.
